(19) Europäisches Patentamt
European Patent Office
Office européen des brevets

(11) Veröffentlichungsnummer: **0 437 699 B1**

# (12) EUROPÄISCHE PATENTSCHRIFT

(45) Veröffentlichungstag der Patentschrift: **22.06.94**

(51) Int. Cl.5: **C09B 62/51**, C09B 62/085, D06P 1/38, C07C 317/28

(21) Anmeldenummer: **90121853.7**

(22) Anmeldetag: **15.11.90**

## (54) Phenylazo- oder Naphthylazobenzole mit mehreren reaktiven Gruppen sowie deren Zwischenprodukte.

(30) Priorität: **28.11.89 DE 3939286**

(43) Veröffentlichungstag der Anmeldung:
**24.07.91 Patentblatt 91/30**

(45) Bekanntmachung des Hinweises auf die Patenterteilung:
**22.06.94 Patentblatt 94/25**

(84) Benannte Vertragsstaaten:
**CH DE FR GB IT LI**

(56) Entgegenhaltungen:
**EP-A- 0 174 909**

(73) Patentinhaber: **BASF Aktiengesellschaft**
**Carl-Bosch-Strasse 38**
**D-67063 Ludwigshafen(DE)**

(72) Erfinder: **Siegel, Bernd, Dr.**
**Faberstrasse 39**
**W-6700 Ludwigshafen(DE)**
Erfinder: **Patsch, Manfred, Dr.**
**Fritz-Wendel-Strasse 4**
**W-6706 Wachenheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Reaktivfarbstoffe der Formel I

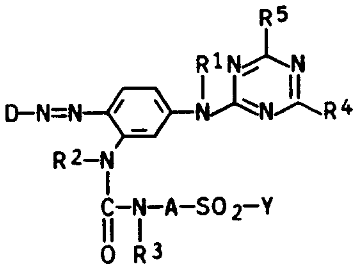

(I) ,

in der

| | |
|---|---|
| $R^1$, $R^2$ und $R^3$ | gleich oder verschieden sind und unabhängig voneinander jeweils Wasserstoff, $C_1$-$C_4$-Alkyl oder Phenyl, |
| $R^4$ | $C_1$-$C_4$-Alkoxy, Phenoxy oder einen Rest der Formel $NL^1L^2$, wobei $L^1$ und $L^2$ gleich oder verschieden sind und unabhängig voneinander jeweils für $C_1$-$C_4$-Alkyl, das gegebenenfalls durch Hydroxy, Hydroxysulfonyl oder Sulfato substituiert ist, oder gegebenenfalls substituiertes Phenyl oder $L^1$ und $L^2$ zusammen mit dem sie verbindenden Stickstoffatom für einen 5- oder 6-gliedrigen gesättigten heterocyclischen Rest, der gegebenenfalls noch weitere Heteroatome aufweisen kann, oder $L^1$ auch für Wasserstoff stehen, |
| $R^5$ | Fluor, Chlor, Brom, $C_1$-$C_4$-Alkylsulfonyl, Phenylsulfonyl oder einen Rest der Formel |

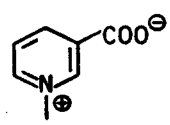

,

| | |
|---|---|
| A | $C_2$-$C_8$-Alkylen, das gegebenenfalls durch 1 bis 3 Sauerstoffatome, Iminogruppen oder $C_1$-$C_4$-Alkyliminogruppen unterbrochen ist, |
| Y | Vinyl oder einen Rest der Formel $-CH_2-CH_2-Q$, wobei Q für eine unter alkalischen Reaktionsbedingungen abspaltbare Gruppe steht, und |
| D | Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder Vinylsulfonyl substituiert sein können, bedeuten, |

neue Phenylendiamine als deren Zwischenprodukte sowie die Verwendung der neuen Farbstoffe zum Färben oder Bedrucken von Hydroxyl- oder Aminogruppen aufweisenden Fasern.

Aus der EP-A-174 909 sind Reaktivfarbstoffe bekannt, die als reaktive Gruppe den Vinylsulfonylrest aufweisen, wobei dieser über eine Ureidogruppe an den Farbstoffrest geknüpft ist.

Aufgabe der vorliegenden Erfindung war es, neue Reaktivfarbstoffe bereitzustellen, die ebenfalls eine Vinylsulfonylgruppe aufweisen, welche über eine Ureidogruppe an den Chromophor gebunden ist, wobei die neuen Farbstoffe günstige anwendungstechnische Eigenschaften aufweisen sollten.

Demgemäß wurden die eingangs näher bezeichneten Reaktivfarbstoffe der Formel I gefunden.

Alle in der obengenannten Formel I auftretenden Alkyl- und Alkylengruppen können sowohl geradkettig als auch verzweigt sein.

Wenn in der obengenannten Formel I substituierte Phenylreste auftreten, so können dabei, sofern nicht anders vermerkt, z.B. $C_1$-$C_4$-Alkyl, $C_1$-$C_4$-Halogenalkyl, $C_1$-$C_4$-Alkoxy, Amino, $C_1$-$C_4$-Mono- oder Dialkylamino, Nitro, Formyl, Cyano, Carboxyl oder Hydroxysulfonyl als Substituenten in Betracht kommen.

Reste $R^1$, $R^2$ und $R^3$ sind z.B. Methyl, Ethyl, Propyl, Isopropyl, Butyl, Isobutyl oder sec-Butyl.

Reste $R^4$ sind z.B. Methoxy, Ethoxy, Propoxy, Isopropoxy, Butoxy, Isobutoxy, sec-Butoxy, Mono- oder Dimethylamino, Mono- oder Diethylamino, Mono- oder Dipropylamino, Mono- oder Diisopropylamino, Mono- oder Dibutylamino, N-Methyl-N-ethylamino, N-(2-Hydroxyethyl)amino, N-(2-Hydroxysulfonylethyl)amino, N-(2-Sulfatoethyl)amino, Mono- oder Diphenylamino, N-Methyl-N-phenylamino, N-Ethyl-N-phenylamino, N-Propyl-N-phenylamino, N-Isopropyl-N-phenylamino, N-Butyl-N-phenylamino, 2- oder 4-Methylphenylamino,

N-Methyl-N-(2- oder 4-methylphenyl)amino, N-Ethyl-N-(2- oder 4-methylphenyl)amino, 2- oder 4-Chlormethylphenylamino, N-Methyl-N-(2-oder 4-chlormethylphenyl)amino, N-Ethyl-N-(2- oder 4-chlormethylphenyl)-amino, 2- oder 4-Trifluormethylphenylamino, N-Methyl-N-(2- oder 4-trifluormethylphenyl)amino, N-Ethyl-N-(2- oder 4-trifluormethylphenyl)amino, 2-Methoxyphenylamino, N-Methyl-N-(2-methoxyphenyl)amino, N-Ethyl-N-(2-methoxyphenyl)amino, 2-Aminophenylamino, N-Methyl-N-(2-aminophenyl)amino, N-Ethyl-N-(2-aminophenyl)amino, 2-Methylaminophenylamino, N-Methyl-N-(2-methylaminophenyl)amino, N-Ethyl-N-(2-methylaminophenyl)amino, 2-Dimethylaminophenylamino, N-Methyl-N-(2-dimethylaminophenyl)amino, N-Ethyl-N-(2-dimethylaminophenyl)amino, 3-Nitrophenylamino, N-Methyl-N-(3-nitrophenyl)amino, N-Ethyl-N-(3-nitrophenyl)amino, 3-Cyanophenylamino, N-Methyl-N-(3-cyanophenyl)amino, N-Ethyl-N-(3-cyanophenylamino), 3-Formylphenylamino, N-Methyl-N-(3-formylphenyl)amino, N-Ethyl-N-(3-formylphenyl)amino, 3-Carboxylphenylamino, N-Methyl-N-(3-carboxylphenyl)amino, N-Ethyl-N-(3-carboxylphenyl)amino, 3-Hydroxysulfonylphenylamino, N-Methyl-N-(3-hydroxysulfonylphenyl)amino, N-Ethyl-N-(3-hydroxysulfonylphenyl)amino, Pyrrolidino, Piperidino, Morpholino, Piperazino oder N-($C_1$-$C_4$-Alkyl)piperazino.

Reste $R^5$ sind z.B. Methylsulfonyl, Ethylsulfonyl, Propylsulfonyl, Isopropylsulfonyl oder Butylsulfonyl.

Reste A sind z.B. $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-CH(CH_3)-CH_2-$, $-CH(CH_3)-CH(CH_3)-$, $-(CH_2)_2-O-(CH_2)_2-$,$-(CH_2)_2-NH-(CH_2)_2-$, $-(CH_2)_2-N(CH_3)-(CH_2)_2-$, $-(CH_2)_2-O-(CH_2)_2-O-(CH_2)_2-$ oder $-(CH_2)_2-O-(CH_2)_2-O-(CH_2)_2-O-(CH_2)_2-$.

Y in Formel I steht u.a. für eine unter alkalischen Reaktionsbedingungen abspaltbare Gruppe. Solche Gruppen sind z.B. Chlor, $OSO_3H$, $SSO_3H$, $OP(O)(OH)_2$, $C_1$-$C_4$-Alkylsulfonyloxy, gegebenenfalls substituiertes Phenylsulfonyloxy, $C_1$-$C_4$-Alkanoyloxy, $C_1$-$C_4$-Dialkylamino,

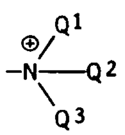 An$^\ominus$ , 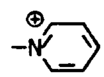 An$^\ominus$ , 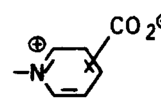 oder 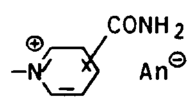 An$^\ominus$ ,

wobei $Q^1$, $Q^2$ und $Q^3$ gleich oder verschieden sind und unabhängig voneinander jeweils die Bedeutung von $C_1$-$C_4$-Alkyl oder Benzyl und An$^\ominus$ jeweils die Bedeutung eines Anions besitzen. (Geeignete Anionen sind z.B. Fluorid, Chlorid, Bromid, Iodid, Mono-, Di- oder Trichloracetat, Methylsulfonat, Phenylsulfonat oder 2- oder 4-Methylphenylsulfonat.)

Reste D sind z.B. 2,4-Dihydroxysulfonylphenyl, 2,5-Dihydroxysulfonylphenyl, 2,5-Dihydroxysulfonyl-4-methylphenyl, 2,5-Dihydroxysulfonyl-6-chlorphenyl, 3,6,8-Trihydroxysulfonylnaphth-2-yl, 4,6,8-Trihydroxysulfonylnaphth-2-yl,1,5-Dihydroxysulfonylnaphth-2-yl oder 1,6-Dihydroxysulfonylnaphth-2-yl.

Bevorzugt sind Reaktivfarbstoffe der Formel I, in der

| | |
|---|---|
| $R^1$, $R^2$ und $R^3$ | jeweils Wasserstoff, |
| $R^4$ | einen Rest der Formel $NL^1L^2$, wobei $L^1$ für $C_1$-$C_4$-Alkyl und $L^2$ für gegebenenfalls substituierter Phenyl stehen, |
| $R^5$ | Fluor oder Chlor, |
| A | $C_2$-$C_4$-Alkylen, das gegebenenfalls durch ein Sauerstoffatom unterbrochen ist, und |
| D | Phenyl oder Naphthyl, wobei diese Reste durch Hydroxysulfonyl ein- bis dreifach substituiert sind, bedeuten und |
| Y | die obengenannte Bedeutung besitzt. |

Insbesondere bevorzugt sind Reaktivfarbstoffe der Formel I, in der

| | |
|---|---|
| $R^4$ | einen Rest der Formel $NL^1L^2$, wobei $L^1$ für $C_1$-$C_4$-Alkyl und $L^2$ für Phenyl stehen, und |
| D | Dihydroxysulfonylphenyl oder Trihydroxysulfonylnaphthyl bedeuten. |

Die neuen Reaktivfarbstoffe der Formel I werden in vorteilhafter Weise erhalten, wenn man auf an sich bekanntem Weg z.B. ein Amin der Formel III

$D-NH_2$ (III),

in der D die obengenannte Bedeutung besitzt, diazotiert und mit einem Phenylendiamin der Formel II

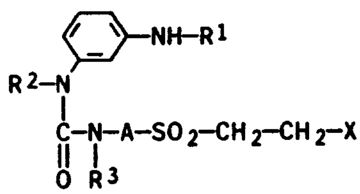

(II),

in der $R^1$, $R^2$, $R^3$ und A jeweils die obengenannte Bedeutung besitzen und X Hydroxy oder Halogen bedeutet, kuppelt.

Der resultierende Azofarbstoff der Formel IV

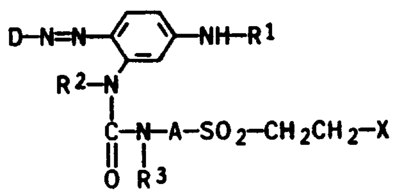

(IV),

in der D, $R^1$, $R^2$, $R^3$, A und X jeweils die obengenannte Bedeutung besitzen, kann dann in bekannter Weise mit Trihalogentriazin und anschließend mit einer Verbindung der Formel V

$R^4$-H (V),

in der $R^4$ die obengenannte Bedeutung besitzt, umgesetzt werden.

Für den Fall, daß X Hydroxy bedeutet, wird schließlich, ebenfalls auf an sich bekanntem Wege, z.B. durch Veresterung mit Schwefelsäure, die Gruppierung $-CH_2CH_2-X$ in den Rest Y (Bedeutung siehe oben) übergeführt.

Die neuen Reaktivfarbstoffe der Formel I eignen sich in vorteilhafter Weise zum Färben oder Bedrucken von Hydroxylgruppen oder Aminogruppen aufweisenden organischen Substraten. Solche Substrate sind beispielsweise Leder oder Fasermaterial, das überwiegend natürliche oder synthetische Polyamide oder natürliche oder regenerierte Cellulose enthält. Vorzugsweise eignen sich die neuen Farbstoffe zum Färben und Bedrucken von Textilmaterial auf der Basis von Baumwolle. Die erfindungsgemäßen Reaktivfarbstoffe können auch für den Ätzdruck verwendet werden, da sie alkalisch ätzbar sind.

Ein weiterer Gegenstand der vorliegenden Erfindung sind Phenylendiamine der Formel II

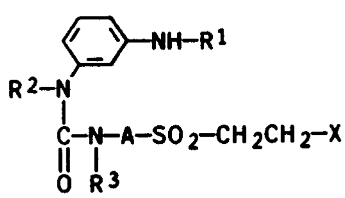

(II),

in der

$R^1$, $R^2$ und $R^3$ gleich oder verschieden sind und unabhängig voneinander jeweils Wasserstoff, $C_1$-$C_4$-Alkyl oder Phenyl,

A $C_2$-$C_8$-Alkylen, das gegebenenfalls durch 1 bis 3 Sauerstoffatome, Iminogruppen oder $C_1$-$C_4$-Alkyliminogruppen unterbrochen ist, und

X Hydroxy oder Halogen bedeuten.

Was die beispielhafte Erläuterung der genannten Reste betrifft, sei auf die voranstehend vorgenommenen Ausführungen verwiesen.

Die neuen Phenylendiamine der Formel II können nach an sich bekannten Methoden erhalten werden. Beispielsweise indem man 3-Nitrophenylisocyanat der Formel VI

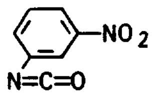

(VI)

mit einem schwefelhaltigen Amin der Formel VII

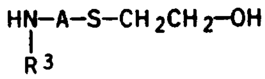

(VII),

in der $R^3$ und A jeweils die obengenannte Bedeutung besitzen, umsetzt und in der resultierenden Thioverbindung der Formel VIII

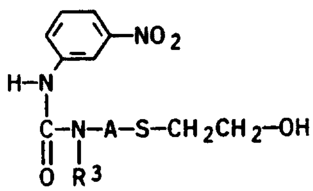

(VIII),

in der $R^3$ und A jeweils die obengenannte Bedeutung besitzen, auf an sich bekanntem Wege zunächst die Thiogruppierung zur Sulfongruppe oxidiert und anschließend die Nitrogruppe zur Aminogruppe reduziert. Mit an sich üblichen Halogenierungsmitteln (z.B. Thionylchlorid, Phosphortrichlorid oder Phosphortribromid) kann dann die Hydroxygruppe durch Halogen ausgetauscht werden.

Die erfindungsgemäßen Phenylendiaminderivate der Formel II sind wertvolle Zwischenprodukte für die Herstellung der neuen Reaktivfarbstoffe der Formel I.

Die folgenden Beispiele sollen die Erfindung näher erläutern.

Darstellung der Zwischenprodukte

Beispiel 1

a) 82 g (0,5 mol) 3-Nitrophenylisocyanat in 500 ml Toluol wurden zu einer Lösung aus 60,5 g (0,5 mol) 2-Amino-2'-hydroxydiethylsulfid und 200 ml Toluol bei einer Temperatur von 30 bis 40 °C getropft. Nach beendeter Reaktion wurde das Lösungsmittel abgezogen, der Rückstand mit 500 ml Wasser und 1,5 g Natriumwolframat x 2 $H_2O$ versetzt und bei ca. 70 °C mit 325 g 30 gew.-%iger Wasserstoffperoxidlösung oxidiert. Das Reaktionsgemisch wurde noch 1,5 Stunden bei dieser Temperatur nachgerührt, anschließend auf ca. 5 °C abgekühlt und vom Niederschlag abfiltriert. Der Filterkuchen wurde mit Natriumbisulfitlösung und Wasser gewaschen. Es resultierten 125 g der Verbindung der Formel

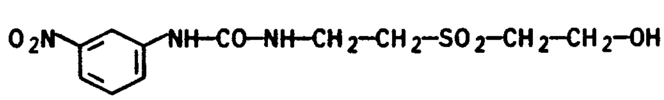

mit dem Schmelzpunkt 174-177 °C. (Die Verbindung kann aus Eisessig umkristallisiert werden.)

b) 110 g (0,35 mol) der in Beispiel 1a) beschriebenen Verbindung wurden in 350 ml N,N-Dimethylformamid gelöst, mit 3,5 g Palladium auf Kohle (10 gew.-%ig) versetzt, auf 60 °C erwärmt und mit 23,3 l Wasserstoff hydriert. Anschließend wurde vom Katalysator abgetrennt, das Lösungsmittel abgezogen und der ölige Rückstand mit 100 ml Aceton zur Kristallisation gebracht. Es verblieben 80 g der Verbindung der Formel

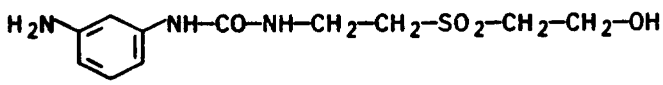

mit einem Schmelzpunkt von 127-131 °C.

Analog Beispiel 1 werden die in der folgenden Tabelle 1 aufgeführten Verbindungen erhalten.

Tabelle 1

| Bsp.-Nr. | Verbindung |
|---|---|
| 2 | 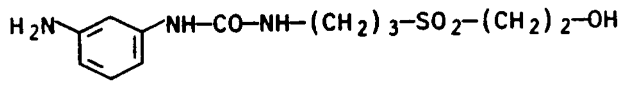 |
| 3 | 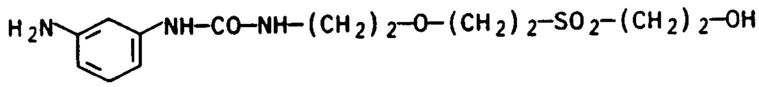 |
| 4 | 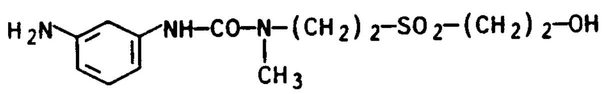 |

Beispiel 5

a) 79,3 g (0,25 mol) des in Beispiel 1a beschriebenen Produktes wurden in 300 ml 1,1,1-Trichlorethan gelöst, mit 29,8 g (0,25 mol) Thionylchlorid bei 30 bis 40 °C versetzt und bis zur Beendigung der Gasentwicklung zum Sieden erhitzt. Anschließend wurde das Lösungsmittel und überschüssiges Thionylchlorid unter vermindertem Druck abdestilliert und der verbleibende Rückstand gegebenenfalls aus Eisessig umkristallisiert. Es verblieben 44,2 g der Verbindung der Formel

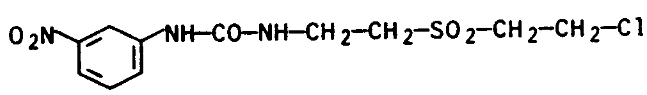

mit dem Schmelzpunkt 126-130 °C.

b) 67 g des in Beispiel 5a) beschriebenen Produktes wurden in 170 ml N,N-Dimethylformamid gelöst und mit Raney-Nickel bei 60 °C hydriert. Nach üblicher Aufarbeitung wurden 47 g einer Verbindung isoliert, die der Formel

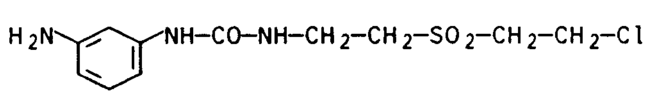

entspricht.

Die in der folgenden Tabelle 2 aufgeführten Verbindungen werden analog Beispiel 5 erhalten.

Tabelle 2

| Bsp.-Nr. | Verbindung |
|---|---|
| 6 | 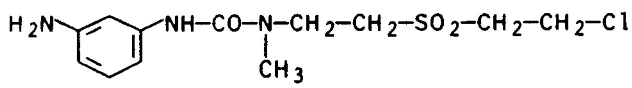 |
| 7 | 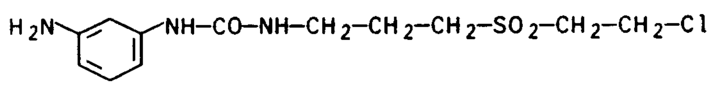 |
| 8 | 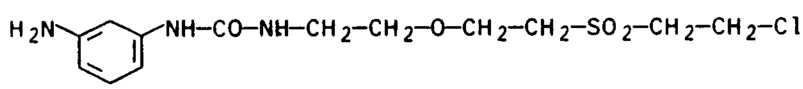 |

Darstellung der Farbstoffe

Beispiel 9

22,1 g (0,1 mol) Anilin-2,4-disulfonsäure wurden in 200 ml Wasser salzsauer diazotiert und die Diazoniumsalzsuspension zu einer Lösung von 28,7 g (0,1 mol) des Produkts aus Beispiel 1b) in 200 ml Wasser, wobei der pH-Wert der Lösung 3 betrug, bei 5 bis 10 °C gegeben. Nach beendeter Kupplung (bei pH = 5 bis 6) wurde die Farbstofflösung bei 0 bis 5 °C mit einer Suspension aus 18,4 g (0,1 mol) Cyanurchlorid und 200 ml Eiswasser versetzt und innerhalb von 1,5 Stunden bei pH = 5 bis 6 auf Raumtemperatur gebracht. Nach beendeter Umsetzung wurde das Reaktionsgemisch mit 12,5 g (0,1 mol) salzsauer gelöstem N-Ethylanilin vermischt, der pH-Wert zwischen 6 und 7 gehalten und bis zur vollständigen Kondensation auf 30 bis 35 °C erwärmt. Anschließend wurde das Lösungsmittel verdampft und ein Farbstoff der Formel

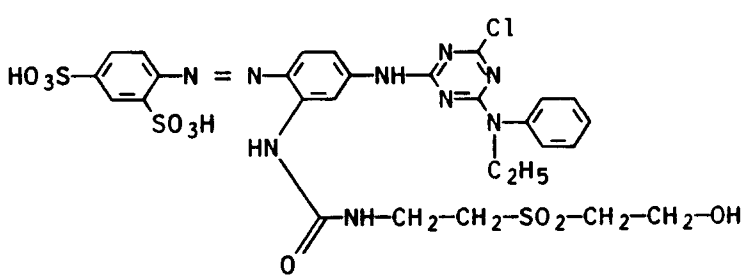

zwischenisoliert.

75 g dieses Farbstoffes wurden bei 20 bis 30 °C portionsweise in 300 g Schwefelsäure-Monohydrat eingetragen. Es wurde 1,5 Stunden bei 40 °C nachgerührt und anschließend auf 1 kg Eis gefällt. Der resultierende Reaktivfarbstoff entspricht der Formel

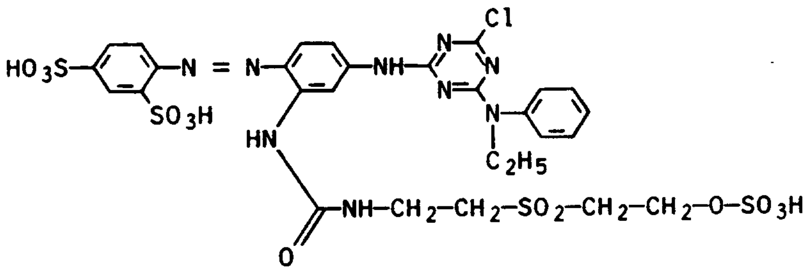

und färbt Baumwolle in goldgelbem Ton mit sehr guten Licht- und Naßechtheiten.

In analoger Weise werden die in Tabelle 3 aufgeführten Farbstoffe der Formel

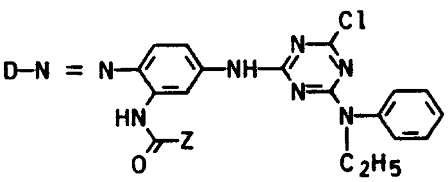

erhalten.

Tabelle 3

| Bsp.-Nr. | D | Z | Farbton auf Baumwolle |
|---|---|---|---|
| 10 | 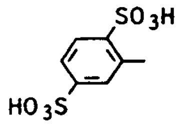 | $NH(CH_2)_2-SO_2-(CH_2)_2-O-SO_3H$ | goldgelb |
| 11 | 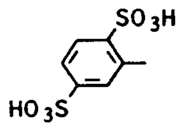 | $NH(CH_2)_3-SO_2-(CH_2)_2-O-SO_3H$ | goldgelb |
| 12 | 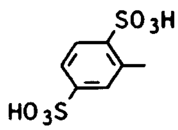 | $\underset{CH_3}{N}(CH_2)_2-SO_2-(CH_2)_2-O-SO_3H$ | goldgelb |
| 13 | 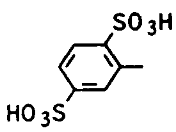 | $NH(CH_2)_2-O-(CH_2)_2-SO_2-(CH_2)_2-O-SO_3H$ | goldgelb |
| 14 | 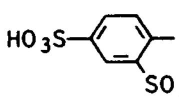 | $NH(CH_2)_3-SO_2-(CH_2)_2-O-SO_3H$ | goldgelb |
| 15 | 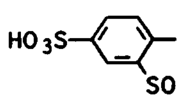 | $NH(CH_2)_2-O-(CH_2)_2-SO_2-(CH_2)_2-O-SO_3H$ | goldgelb |
| 16 | 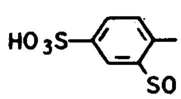 | $\underset{CH_3}{N}-(CH_2)_2-SO_2-(CH_2)_2-O-SO_3H$ | goldgelb |
| 17 | 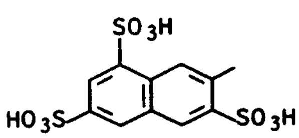 | $NH(CH_2)_2-SO_2-(CH_2)_2-O-SO_3H$ | goldgelb |

Tabelle 3

| Bsp.-Nr. | D | Z | Farbton auf Baumwolle |
|---|---|---|---|
| 18 | 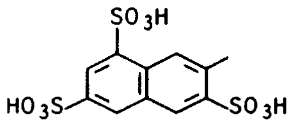 | $NH(CH_2)_3-SO_2-(CH_2)_2-O-SO_3H$ | goldgelb |
| 19 | 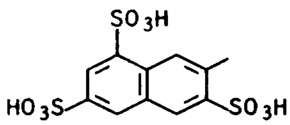 | $NH(CH_2)_2-O-(CH_2)_2-SO_2-(CH_2)_2-O-SO_3H$ | goldgelb |
| 20 | 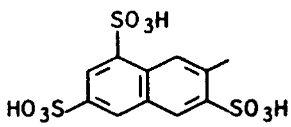 | $N(CH_3)-(CH_2)_2-SO_2-(CH_2)_2-O-SO_3H$ | goldgelb |
| 21 | 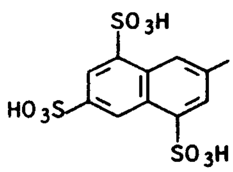 | $NH(CH_2)_2-SO_2-(CH_2)_2-O-SO_3H$ | goldgelb |
| 22 | 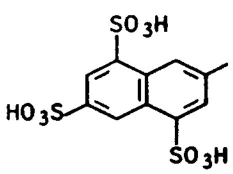 | $NH(CH_2)_3-SO_2-(CH_2)_2-O-SO_3H$ | goldgelb |
| 23 | 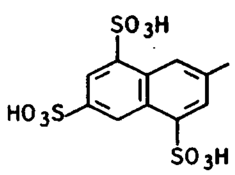 | $NH(CH_2)_2-O-(CH_2)_2-SO_2-(CH_2)_2-O-SO_3H$ | goldgelb |
| 24 | 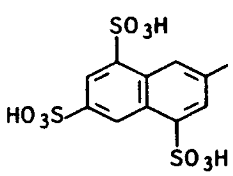 | $N(CH_3)-(CH_2)_2-SO_2-(CH_2)_2-O-SO_3H$ | goldgelb |

Beispiel 25

22,1 g (0,1 mol) Anilin-2,4-disulfonsäure wurden in 200 ml Wasser salzsauer diazotiert und die Diazoniumsalzsuspension zu einer Lösung von 28,7 g (0,1 mol) des Produktes aus Beispiel 1b in 250 ml Wasser, wobei der pH-Wert der Lösung 3 betrug, bei 5 bis 10 °C gegeben. Nach beendeter Kupplung bei

pH = 5 bis 6 wurde das Lösungsmittel eingedampft und 108,5 g elektrolythaltiges Pulver, das den Farbstoff der Formel

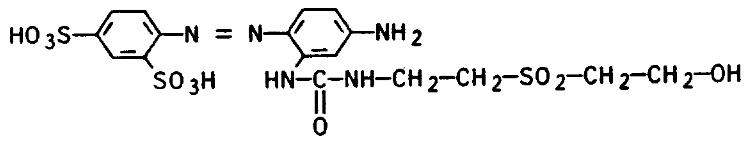

($\lambda_{max}$ = 441 nm)

enthielt, isoliert.

108 g dieses Pulvers wurden in 400 g Schwefelsäure-Monohydrat portionsweise bis zu einer Innentemperatur von 40 °C eingetragen und ca. 8 Stunden bei Raumtemperatur gerührt. Nach vollständiger Umsetzung wurde die Reaktionslösung auf 1000 g Eis gefällt, mit Natriumhydrogencarbonat auf einen pH-Wert von 4 bis 5 gestellt, vom ausgeschiedenen Natriumsulfat abgetrennt und mit einer Suspension aus 18,4 g (0,1 mol) 1,3,5-Trichlortriazin in 200 ml Eiswasser bei 0 bis 5 °C versetzt. Die Kondensationsreaktion wurde bei einem pH-Wert von 5 bis 6 und 5 bis 10 °C durchgeführt. Anschließend versetzte man das Reaktionsgemisch mit 12,5 g (0,1 mol) salzsauer gelöstem N-Ethylanilin und hielt einen pH-Wert von 6 und 20 bis 30 °C bis zum Reaktionsende ein. Durch Zugabe von Kaliumchlorid wurde ein salzhaltiger Farbstoff isoliert, der in Form der freien Säure der Formel

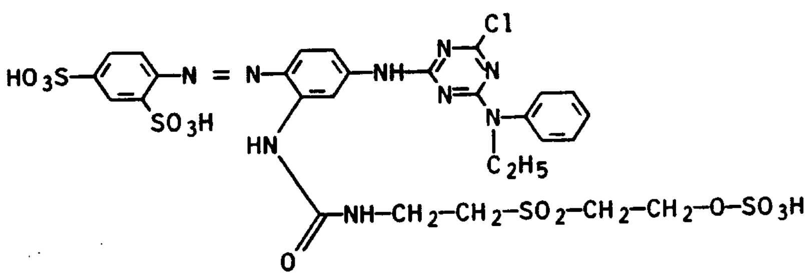

entspricht. Er färbt Cellulosefasern in goldgelbem Ton mit sehr guten Lichtechtheiten und guten Naßechtheiten.

(Die in der obigen Tabelle 3 aufgeführten Verbindungen können auch analog Beispiel 25 hergestellt werden.)

Beispiel 26

22,1 g (0,1 mol) salzsauer diazotierte Anilin-2,4-disulfonsäure wurden mit 30,6 g (0,1 mol) des Produkts aus Beispiel 5b) bei einem pH-Wert von 4 bis 5 gekuppelt und anschließend bei 5 bis 10 °C mit 18,4 g (0,1 mol) Cyanurchlorid bei einem pH-Wert von 5 bis 6 kondensiert. Danach wurde das Reaktionsgemisch bei 20 bis 30 °C mit 12,5 g (0,1 mol) salzsauer gelöstem N-Ethylanilin versetzt und bis zur vollständigen Umsetzung bei einem pH-Wert von 5 bis 6 gehalten. Durch Aussalzen mittels Kaliumchlorid wurde ein salzhaltiger Farbstoff isoliert, der in Form der freien Säure der Formel

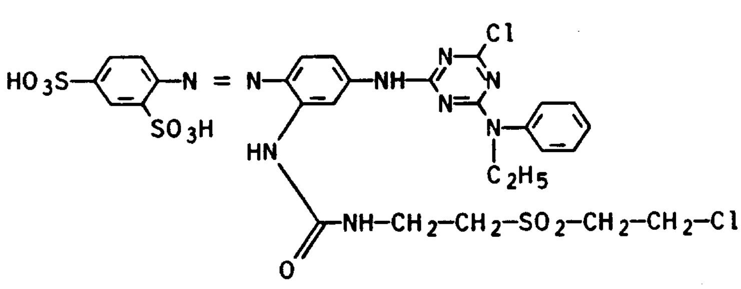

entspricht. Er färbt Baumwolle in goldgelbem Ton mit guten Echtheiten.

Analog Beispiel 26 werden die in Tabelle 4 angeführten Farbstoffe der Formel

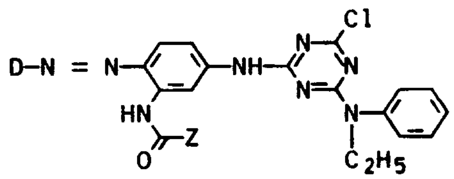

erhalten.

Tabelle 4

| Bsp.-Nr. | D | Z | Farbton auf Baumwolle |
|---|---|---|---|
| 27 | 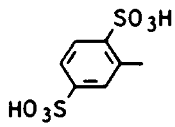 | $NH(CH_2)_2-SO_2-(CH_2)_2-Cl$ | goldgelb |
| 28 | 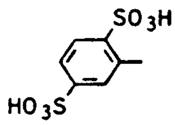 | $NH(CH_2)_3-SO_2-(CH_2)_2-Cl$ | goldgelb |
| 29 | 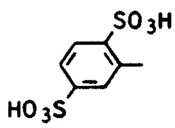 | $N(CH_2)_2-SO_2-(CH_2)_2-Cl$<br>$\vert$<br>$CH_3$ | goldgelb |
| 30 | 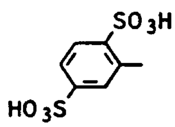 | $NH(CH_2)_2-O-(CH_2)_2-SO_2-(CH_2)_2-Cl$ | goldgelb |
| 31 | 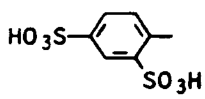 | $NH(CH_2)_3-SO_2-(CH_2)_2-Cl$ | goldgelb |
| 32 | 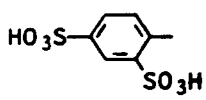 | $NH(CH_2)_2-O-(CH_2)_2-SO_2-(CH_2)_2-Cl$ | goldgelb |
| 33 | 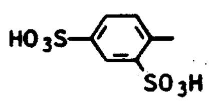 | $N-(CH_2)_2-SO_2-(CH_2)_2-Cl$<br>$\vert$<br>$CH_3$ | goldgelb |
| 34 | 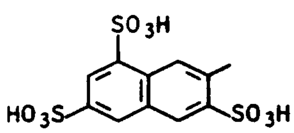 | $NH(CH_2)_2-SO_2-(CH_2)_2-Cl$ | goldgelb |

| Bsp.-Nr. | D | Z | Farbton auf Baumwolle |
|---|---|---|---|
| 35 | 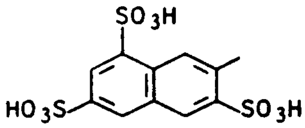 | $NH(CH_2)_3-SO_2-(CH_2)_2-Cl$ | goldgelb |
| 36 | 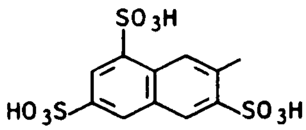 | $NH(CH_2)_2-O-(CH_2)_2-SO_2-(CH_2)_2-Cl$ | goldgelb |
| 37 | 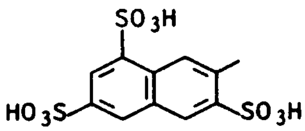 | $N(CH_3)-(CH_2)_2-SO_2-(CH_2)_2-Cl$ | goldgelb |
| 38 | 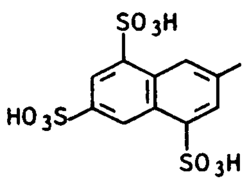 | $NH(CH_2)_2-SO_2-(CH_2)_2-Cl$ | goldgelb |
| 39 | 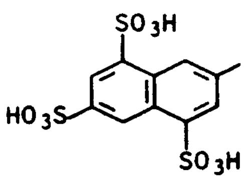 | $NH(CH_2)_3-SO_2-(CH_2)_2-Cl$ | goldgelb |
| 40 | 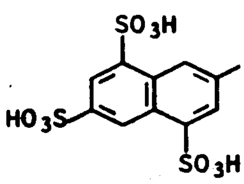 | $NH(CH_2)_2-O-(CH_2)_2-SO_2-(CH_2)_2-Cl$ | goldgelb |
| 41 | 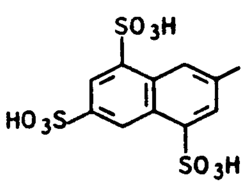 | $N(CH_3)-(CH_2)_2-SO_2-(CH_2)_2-O-Cl$ | goldgelb |

Beispiel 42

a) 108 g salzhaltiger Farbstoff der Formel

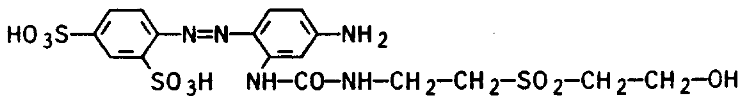

wurden in 400 g Schwefelsäure-Monohydrat bis zu einer Innentemperatur von 40 °C eingetragen und anschließend für 8 Stunden bei Raumtemperatur gerührt. Danach wurde mit 800 ml Eiswasser hydrolisiert und das Produkt mit Aceton ausgefällt. Es verblieben 62,7 g einer Verbindung der Formel

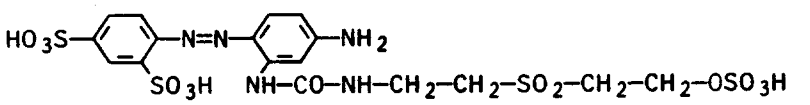

($\lambda_{max}$ = 455 nm)

b) 31,6 g (0,05 mol) des in a) beschriebenen Azofarbstoffs wurden mit 300 ml Wasser angerührt und mit jeweils der äquivalenten Menge an Cyanurchlorid und N-Ethylanilin analog Beispiel 25 kondensiert.

c) Zur Überführung in die Vinylsulfonverbindung wurde die in Beispiel 42b erhaltene Lösung bei ca. 30 °C mit 2 n Natronlauge bis zur vollständigen Eliminierung auf pH = 9 bis 10 gestellt und danach neutralisiert. Durch Aussalzen mit Kaliumchlorid wurde ein salzhaltiger Farbstoff isoliert, der in Form der freien Säure der Formel

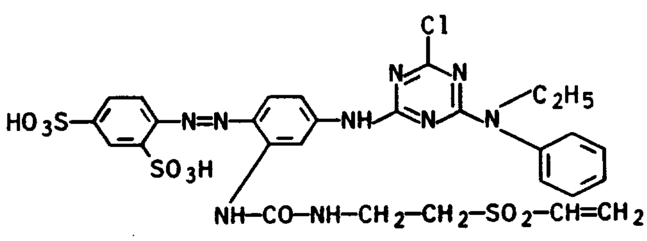

($\lambda_{max}$ = 387 nm)

entspricht. Er färbt Baumwolle in goldgelbem Ton mit guten Echtheiten.

Diese Vinylverbindung ist in entsprechender Weise auch aus dem in Beispiel 26 beschriebenen Farbstoff darstellbar.

(In analoger Weise können auch die in den Tabellen 3 und 4 aufgeführten Beispiele als Vinylverbindungen erhalten werden.)

**Patentansprüche**

**1.** Reaktivfarbstoffe der Formel I

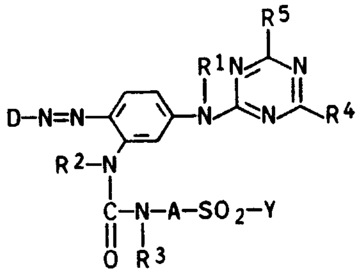

(I) ,

in der

| | |
|---|---|
| $R^1$, $R^2$ und $R^3$ | gleich oder verschieden sind und unabhängig voneinander jeweils Wasserstoff, $C_1$-$C_4$-Alkyl oder Phenyl, |
| $R^4$ | $C_1$-$C_4$-Alkoxy, Phenoxy oder einen Rest der Formel $NL^1L^2$, wobei $L^1$ und $L^2$ gleich oder verschieden sind und unabhängig voneinander jeweils für $C_1$-$C_4$-Alkyl, das gegebenenfalls durch Hydroxy, Hydroxysulfonyl oder Sulfato substituiert ist, oder gegebenenfalls substituiertes Phenyl oder $L^1$ und $L^2$ zusammen mit dem sie verbindenden Stickstoffatom für einen 5- oder 6-gliedrigen gesättigten heterocyclischen Rest, der gegebenenfalls noch weitere Heteroatome aufweisen kann, oder $L^1$ auch für Wasserstoff stehen, |
| $R^5$ | Fluor, Chlor, Brom, $C_1$-$C_4$-Alkylsulfonyl, Phenylsulfonyl oder einen Rest der Formel |

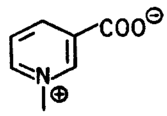

,

| | |
|---|---|
| A | $C_2$-$C_8$-Alkylen, das gegebenenfalls durch 1 bis 3 Sauerstoffatome, Iminogrupppen oder $C_1$-$C_4$-Alkyliminogruppen unterbrochen ist, |
| Y | Vinyl oder einen Rest der Formel $-CH_2-CH_2-Q$, wobei Q für eine unter alkalischen Reaktionsbedingungen abspaltbare Gruppe steht, und |
| D | Phenyl oder Naphthyl, wobei diese Reste ein- oder mehrfach durch Hydroxysulfonyl, Carboxyl, $C_1$-$C_4$-Alkoxycarbonyl, Cyano, $C_1$-$C_4$-Alkyl, $C_1$-$C_4$-Alkoxy, Halogen, Nitro oder vinylsulfonyl substituiert sein können, bedeuten. |

**2.** Reaktivfarbstoffe nach Anspruch 1, dadurch gekennzeichnet, daß

| | |
|---|---|
| $R^1$, $R^2$ und $R^3$ | jeweils Wasserstoff, |
| $R^4$ | einen Rest der Formel $NL^1L^2$, wobei $L^1$ für $C_1$-$C_4$-Alkyl und $L^2$ für gegebenenfalls substituiertes Phenyl stehen, |
| $R^5$ | Fluor oder Chlor, |
| A | $C_2$-$C_4$-Alkylen, das gegebenenfalls durch ein Sauerstoffatom unterbrochen ist, und |
| D | Phenyl oder Naphthyl, wobei diese Reste durch Hydroxysulfonyl ein- bis dreifach substituiert sind, bedeuten und |
| Y | die in Anspruch 1 genannte Bedeutung besitzt. |

**3.** Phenylendiamine der Formel II

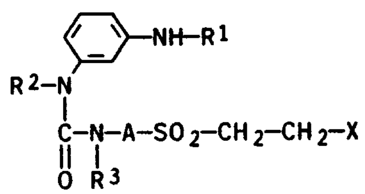

(II),

in der

| | |
|---|---|
| $R^1$, $R^2$ und $R^3$ | gleich oder verschieden sind und unabhängig voneinander jeweils Wasserstoff, $C_1$-$C_4$-Alkyl oder Phenyl, |
| A | $C_2$-$C_8$-Alkylen, das gegebenenfalls durch 1 bis 3 Sauerstoffatome, Iminogruppen oder $C_1$-$C_4$-Alkyliminogruppen unterbrochen ist, und |
| X | Hydroxy oder Halogen bedeuten. |

**4.** Verwendung der Reaktivfarbstoffe gemäß Anspruch 1 zum Färben oder Bedrucken von Hydroxyl- oder Aminogruppen aufweisenden organischen Substraten.

## Claims

**1.** A reactive dye of the formula

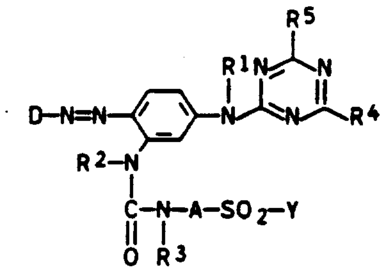

(I)

where

| | |
|---|---|
| $R^1$, $R^2$ and $R^3$ | are identical or different and each is independently of the others hydrogen, $C_1$-$C_4$-alkyl or phenyl, |
| $R^4$ | is $C_1$-$C_4$-alkoxy, phenoxy or a radical of the formula $NL^1L^2$, where $L^1$ and $L^2$ are identical or different and each is independently of the other $C_1$-$C_4$-alkyl, which may be substituted by hydroxyl, hydroxysulfonyl or sulfato, or substituted or unsubstituted phenyl, or $L^1$ and $L^2$ together combine with the nitrogen atom joining them to form a 5- or 6-membered saturated heterocyclic radical which may contain further hetero atoms, or $L^1$ may also be hydrogen, |
| $R^5$ | is fluorine, chlorine, bromine, $C_1$-$C_4$-alkylsulfonyl, phenylsulfonyl or a radical of the formula 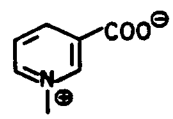 |
| A | is $C_2$-$C_8$-alkylene, which may be interrupted by from 1 to 3 oxygen atoms, imino groups or $C_1$-$C_4$-alkylimino groups, |
| Y | is vinyl or a radical of the formula $-CH_2-CH_2-Q$, where Q is a group which is detachable under alkaline reaction conditions, and |
| D | is phenyl or naphthyl, which may each be monosubstituted or polysubstituted by hydroxysulfonyl, carboxyl, $C_1$-$C_4$-alkoxycarbonyl, cyano, $C_1$-$C_4$-alkyl, $C_1$-$C_4$-alkoxy, halogen, nitro or vinylsulfonyl. |

**2.** A reactive dye as claimed in claim 1, wherein

| | |
|---|---|
| $R^1$, $R^2$ and $R^3$ | are each hydrogen, |
| $R^4$ | is a radical of the formula $NL^1L^2$, where $L^1$ is $C_1$-$C_4$-alkyl and $L^2$ is substituted or unsubstituted phenyl, |
| $R^5$ | is fluorine or chlorine, |
| A | is $C_2$-$C_4$-alkylene, which may be interrupted by an oxygen atom, |
| D | is phenyl or naphthyl, which may both be monosubstituted, disubstituted or trisubstituted by hydroxysulfonyl, and |
| Y | is as defined in claim 1. |

**3.** A phenylenediamine of the formula II

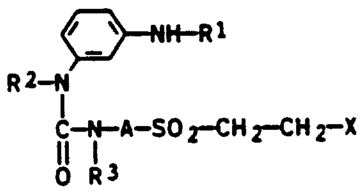

(II)

where

| | |
|---|---|
| $R^1$, $R^2$ and $R^3$ | are identical or different and each is independently of the others hydrogen, $C_1$-$C_4$-alkyl or phenyl, |
| A | is $C_2$-$C_8$-alkylene, which may be interrupted by from 1 to 3 oxygen atoms, imino groups or $C_1$-$C_4$-alkylimino groups, and |
| X | is hydroxyl or halogen. |

**4.** The use of a reactive dye as claimed in claim 1 for dyeing or printing a hydroxyl- or amino-containing organic substrate.

**Revendications**

**1.** Colorants réactifs de formule I

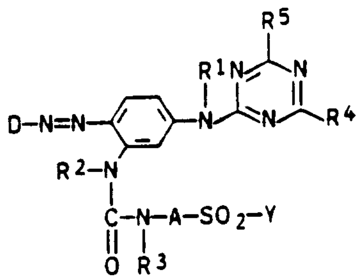

(I) ,

dans laquelle

| | |
|---|---|
| $R^1$, $R^2$ et $R^3$ | sont identiques ou différents et représentent chacun indépendamment l'un de l'autre un atome d'hydrogène, un groupe alkyle en $C_1$-$C_4$ ou phényle, |
| $R^4$ | représente un groupe alcoxy en $C_1$-$C_4$, phénoxy ou un reste de formule $NL^1L^2$, $L^1$ et $L^2$ étant identiques ou différents et représentant chaque fois indépendamment l'un de l'autre un groupe alkyle en $C_1$-$C_4$, qui peut être éventuellement substitué par un groupe hydroxy, hydroxysulfonyle ou sulfato, ou un groupe phényle éventuellement substitué, ou $L^1$ et $L^2$ représentent ensemble, avec l'atome d'azote qui leur est relié, un reste hétérocyclique saturé à 5 ou 6 membres, qui peut éventuellement présenter encore d'autres hétéroatomes, ou $L^1$ représente aussi un atome d'hydrogène, |
| $R^5$ | est mis pour un atome de fluor, de chlore, de brome, un groupe alkylsulfonyle en $C_1$-$C_4$, phénylsulfonyle ou pour un reste de formule 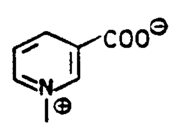 , |
| A | représente un groupe alkylène en $C_2$-$C_8$, qui peut être éventuellement interrompu par 1 à 3 atomes d'oxygène, par des groupes imino ou alkylimino en $C_1$-$C_4$, |
| Y | est un groupe vinyle ou un reste de formule $-CH_2-CH_2-Q$, Q étant mis pour un groupe pouvant être arraché dans des conditions réactionnelles alcalines, et |
| D | représente un groupe phényle ou naphtyle, qui peut être substitué une ou plusieurs |

fois par des groupes hydroxysulfonyle, carboxyle, alcoxycarbonyle en $C_1$-$C_4$, cyano, alkyle en $C_1$-$C_4$, alcoxy en $C_1$-$C_4$, halogéno, nitro ou vinylsulfonyle.

**2.** Colorants réactifs selon la revendication 1, caractérisés en ce que

| | |
|---|---|
| $R^1$, $R^2$ et $R^3$ | représentent chaque fois un atome d'hydrogène, |
| $R^4$ | représente un reste de formule $NL^1L^2$, $L^1$ étant mis pour un groupe alkyle en $C_1$-$C_4$ et $L^2$ pour un groupe phényle éventuellement substitué, |
| $R^5$ | est un atome de fluor ou de chlore, |
| A | est un groupe alkylène en $C_2$-$C_4$, qui peut être éventuellement interrompu par un atome d'oxygène, et |
| D | est un groupe phényle ou naphtyle, qui peut être substitue une à trois fois par un groupe hydroxysulfonyle, et |
| Y | a la signification indiquée dans la revendication 1. |

**3.** Phénylènediamines de formule II

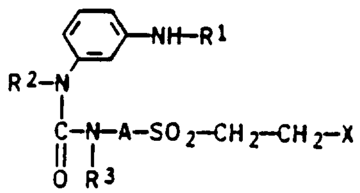

(II),

dans laquelle

| | |
|---|---|
| $R^1$ $R^2$ et $R^3$ | sont identiques ou différents et représentent chaque fois indépendamment l'un de l'autre un atome d'hydrogène, un groupe alkyle en $C_1$-$C_4$ ou phényle, |
| A | représente un groupe alkylène en $C_2$-$C_8$, qui est éventuellement interrompu par 1 à 3 atomes d'oxygène, par des groupes imino ou alkylimino en $C_1$-$C_4$, et |
| X | représente un groupe hydroxy ou halogéno. |

**4.** Utilisation des colorants réactifs selon la revendication 1 pour la teinture ou l'impression de substrats organiques présentant des groupes hydroxyle ou amino.